(19) Europäisches Patentamt
European Patent Office
Office européen des brevets

(11) EP 4 796 215 A1

(12) **EUROPEAN PATENT APPLICATION**
published in accordance with Art. 153(4) EPC

(43) Date of publication:
**26.08.2026 Bulletin 2026/35**

(21) Application number: **24878850.7**

(22) Date of filing: **30.09.2024**

(51) International Patent Classification (IPC):
***B01J 29/00*** *(2006.01)* ***B01J 37/04*** *(2006.01)*
***C07D 307/36*** *(2006.01)*

(52) Cooperative Patent Classification (CPC):
**B01J 29/00; B01J 29/06; B01J 29/072; B01J 29/70; B01J 37/04; C01B 39/04; C07D 307/36; C07D 307/50**

(86) International application number:
**PCT/CN2024/122744**

(87) International publication number:
**WO 2025/082206 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.10.2023 CN 202311361864**
**19.10.2023 CN 202311359263**

(71) Applicants:
- **China Petroleum & Chemical Corporation**
  **Beijing 100728 (CN)**
- **Sinopec (Shanghai) Research Institute of Petrochemical Technology Co., Ltd.**
  **Shanghai 201208 (CN)**

(72) Inventors:
- **YANG, Weimin**
  **Shanghai 201208 (CN)**
- **LI, Xiangcheng**
  **Shanghai 201208 (CN)**
- **WANG, Zhendong**
  **Shanghai 201208 (CN)**
- **HAN, Xiao**
  **Shanghai 201208 (CN)**
- **FENG, Xinqiang**
  **Shanghai 201208 (CN)**
- **QU, Hongyu**
  **Shanghai 201208 (CN)**
- **LIU, Chuang**
  **Shanghai 201208 (CN)**

(74) Representative: **Hoffmann Eitle**
**Patent- und Rechtsanwälte PartmbB**
**Arabellastraße 30**
**81925 München (DE)**

(54) **NON-PRECIOUS METAL CATALYST BASED ON SILICON GERMANIUM MOLECULAR SIEVE, PREPARATION METHOD THEREFOR AND USE THEREOF**

(57) The present disclosure is directed to a catalyst, and a preparation method and use thereof. The catalyst is a Ni-SCM-X molecular sieve catalyst, wherein X is 14 or 15; Ni comprises metallic Ni and $Ni^{2+}$, wherein the molar ratio of $Ni/Ni^{2+}$ is in a range of 0.02-0.15. The catalyst can be used in the reaction for preparing 2,5-dimethylfuran from 5-hydroxymethylfurfural, and has the characteristics of high conversion of 5-hydroxymethylfurfural and high selectivity for the product 2,5-dimethylfuran under mild reaction conditions, as well as high stability of the catalyst upon recycling.

50 nm

FIG. 5A

EP 4 796 215 A1

## Description

## TECHNICAL FIELD

The present disclosure relates to the field of catalytic chemistry, and specifically, relates to a non-noble metal catalyst based on a silicon-germanium molecular sieve and a preparation method and use thereof, and particularly to a catalyst for catalyzing the conversion of 5-hydroxymethylfurfural and/or 5-(C1-C20 alkoxymethyl)furfural to prepare 2,5-dimethylfuran, and a preparation method and use thereof.

## BACKGROUND ART

Due to the gradual depletion of fossil resources and the increasing demand for chemicals and energy in today's society, the preparation of high value-added chemicals and fuels from renewable biomass resources has become one of the research hotspots in the field of new energy. Biomass, as a sustainable and renewable resource, has the characteristics of being abundant, widely distributed, low-cost, and capable of achieving a low-carbon cycle. It can meet society's demand for fuels and chemicals, thereby achieving a transition from non-renewable fossil resources to renewable biomass resources. Several pathways for the synthesis of bio-based para-xylene (PX) have been reported, including, for example, the preparation of PX by biomass pyrolysis or reforming; the catalytic conversion of small molecule compounds derived from biomass to prepare PX, such as the preparation of PX from bio-based ethanol, the preparation of PX from cellulose via isobutanol, or the preparation of PX via the 2,5-dimethylfuran (DMF) route starting from the "sugar platform", etc. Among these, the route via 2,5-dimethylfuran to PX is one of the most deeply and widely studied bio-based synthesis pathways, offering advantages such as high selectivity and low separation energy consumption.

DMF is mainly prepared by the hydrogenolysis of 5-hydroxymethylfurfural (HMF). When hydrogen gas is used as the hydrogen source, commonly used catalysts include supported noble metals, such as ruthenium (Ru), platinum (Pt), and palladium (Pd), as well as transition metals, such as nickel (Ni), copper (Cu), cobalt (Co), etc. CN114832833A reports a $Ni_3Fe$ catalyst supporting single atom noble metal, wherein 0.5 wt% of HMF was dissolved in 60 mL of THF solvent, and the $Ni_3Fe$ catalyst was added according to an HMF/Pt molar ratio of 100/1. After reacting at 160°C under 1 MPa $H_2$ for 90 minutes, HMF was completely converted, and the DMF yield was as high as 99.2%. CN108654693B reports a solid catalytic material obtained by using a porous metal-organic framework as a support, introducing elemental palladium by an impregnation-reduction method, and then treating with a polydimethylsiloxane coating. This material is used for catalyzing the conversion of hexose (especially hexosan) into 2,5-dimethylfuran; for example, when fructose was used as a substrate, after reacting at 110°C for 2.5 h, fructose was completely converted, and the 2,5-dimethylfuran yield reached 93%. In the presence of noble metal catalysts, the production of DMF from HMF usually yields satisfactory results. However, the low recyclability and high cost of noble metals severely hinder their commercial application. CN106279075B reports a heterogeneous iron-based catalyst for catalyzing the preparation of DMF from HMF. In Example 10, after reacting at 200°C under 1 MPa $H_2$ for 12 h, the HMF conversion was 100% and the DMF yield was 64.9%. The catalytic efficiency of this iron-based catalyst needs further improvement.

In summary, the prior art suffers from problems such as high catalyst cost and low catalytic efficiency.

## SUMMARY

The technical problem to be solved by the present disclosure is the problems existing in the prior art, such as high catalyst cost and low catalyst catalytic efficiency. The present disclosure provides a catalyst and a preparation method and use thereof. The catalyst is used for catalyzing the conversion of 5-hydroxymethylfurfural (HMF) and/or 5-(C1-C20 alkoxymethyl)furfural to prepare 2,5-dimethylfuran. When used in the reaction for preparing 2,5-dimethylfuran, the catalyst offers the advantages of high conversion of 5-hydroxymethylfurfural and/or 5-(C1-C20 alkoxymethyl)furfural and high selectivity for the product 2,5-dimethylfuran under mild reaction conditions, as well as high stability of the catalyst upon recycling.

The present disclosure provides a Ni-SCM-X molecular sieve catalyst, wherein X is 14 or 15; wherein Ni comprises metallic Ni and $Ni^{2+}$, wherein the molar ratio of Ni/$Ni^{2+}$ is in a range of 0.02-0.15, preferably 0.05-0.12.

In some embodiments, the catalyst comprises nickel particles, wherein the mean particle size of the nickel particles is in a range of 2.0-8.0 nm, preferably 3.0-7.0 nm, more preferably 3.0-6.0 nm. In some embodiments, in the catalyst, based on the mass of the SCM-X molecular sieve, the content of Ni, calculated as elemental Ni, is in a range of 0.2 wt%-5.0 wt%, preferably 0.5 wt%-3.0 wt%. In some embodiments, in the Ni-SCM-X molecular sieve catalyst, the Si/Ge molar ratio of the SCM-X molecular sieve is in a range of 1.5-8, preferably 2-7. In some embodiments, the total specific surface area of the Ni-SCM-X molecular sieve catalyst is in a range of 90-370 $m^2 \cdot g^{-1}$, preferably 100-350 $m^2 \cdot g^{-1}$; and/or the total acid amount of the Ni-SCM-X molecular sieve catalyst is in a range of 130-220 $\mu mol \cdot g^{-1}$, preferably 150-200 $\mu mol \cdot g^{-1}$; and/or the Brønsted acid/Lewis acid ratio of the Ni-SCM-X molecular sieve catalyst is in a range of 0.3-0.8, preferably

0.4-0.6; and/or, the Ni-SCM-X molecular sieve catalyst is used in a reaction for preparing 2,5-dimethylfuran from 5-hydroxymethylfurfural and/or 5-(C1-C20 alkoxymethyl)furfural.

The present disclosure also provides a method for preparing the above Ni-SCM-X molecular sieve catalyst, comprising: mixing an SCM-X molecular sieve with a nickel-containing precursor to obtain a mixture, and drying (optional), calcining and reducing the obtained mixture to obtain the Ni-SCM-X molecular sieve catalyst.

In some embodiments, the nickel-containing precursor is selected from nickel precursors containing an organic ligand, preferably the organic ligand is selected from organic ligands containing a five-membered carbocyclic ring, a six-membered carbocyclic ring, or an eight-membered carbocyclic ring. In some embodiments, preferably the five-membered carbocyclic ring may be a cyclopentadiene ring, the six-membered carbocyclic ring may be a benzene ring, and the eight-membered carbocyclic ring may be a cyclooctadiene ring. The five-membered carbocyclic ring, six-membered carbocyclic ring or eight-membered carbocyclic ring optionally have substituent(s), for example C1-C4 alkyl group. The nickel-containing precursor is preferably selected from at least one of nickelocene, bis-(1,5-cyclooctadiene) nickel, bis(tetramethylcyclopentadienyl) nickel, 1,2-bis(diphenylphosphino)ethane nickel chloride and bis(triphenylphosphine) nickel chloride, more preferably nickelocene. In some embodiments, the mass ratio of Ni in the nickel-containing precursor to the SCM-X molecular sieve is in a range of 0.2-8.0:100, preferably 0.5-5.0:100. In some embodiments, the mixing is physical mixing; preferably the mixing is grinding or ball milling; and/or the method further comprises drying the mixture before the calcination and reduction; preferably, the conditions for drying include drying at 50-130°C for 2-8 h. In some embodiments, the conditions for calcination include: calcining at 300-650°C for 1-12 h, and the calcination atmosphere is an oxygen-containing gas, preferably the calcination atmosphere is oxygen, air, or a mixed gas of oxygen and air; and/or, the reduction is carried out under a hydrogen atmosphere, the reduction temperature is in a range of 300-500°C, preferably 350-450°C; and the reduction time is in a range of 1-8 h, preferably 2-6 h.

The present disclosure also provides a method for preparing 2,5-dimethylfuran, comprising: in the presence of the above catalyst, subjecting 5-hydroxymethylfurfural and/or 5-(C1-C20 alkoxymethyl)furfural to hydrogenolysis in an organic solvent to prepare 2,5-dimethylfuran.

In some embodiments, the organic solvent comprises at least one of C1-C5 alcohols, cyclic ether compounds, γ-valerolactone, methyl isobutyl ketone, and acetone, preferably the C1-C5 alcohol is selected from methanol, ethanol, n-propanol, n-butanol, isobutanol, isopropanol, isoamyl alcohol, ethylene glycol, propylene glycol and glycerol, and the cyclic ether compound is selected from pyran, 2-methyltetrahydrofuran, 2,5-dimethyltetrahydrofuran, tetrahydrofuran and 1,4-dioxane; preferably the organic solvent comprises at least one of n-butanol, acetone, 1,4-dioxane, γ-valerolactone, methyl isobutyl ketone, and tetrahydrofuran, more preferably the organic solvent comprises tetrahydrofuran. In some embodiments, the hydrogenolysis reaction temperature is in a range of 130-220°C, preferably 150-200°C; the reaction time is in a range of 1-12 h, preferably 2-10 h; and the hydrogen pressure is in a range of 0.5-4 MPa, preferably the hydrogen pressure is in a range of 1-3 MPa; and/or, the mass ratio of 5-hydroxymethylfurfural and/or 5-(C1-C20 alkoxymethyl)furfural to the catalyst is in a range of 0.2-5:1, preferably 0.5-4:1; and/or, the mass ratio of the organic solvent to 5-hydroxymethylfurfural and/or 5-(C1-C20 alkoxymethyl)furfural is in a range of 40-400:1, preferably 50-300:1.

The present disclosure also provides use of the above catalyst or a catalyst prepared by the above preparation method as a catalyst in a reaction for preparing 2,5-dimethylfuran from 5-hydroxymethylfurfural and/or 5-(C1-C20 alkoxymethyl)furfural.

## BRIEF DESCRIPTION OF THE DRAWINGS

FIG. 1 shows an XRD pattern of the Ni-SCM-14 catalyst in Example 1;
FIG. 2 shows an $NH_3$-TPD pattern of the Ni-SCM-14 catalyst in Example 1;
FIG. 3 shows a pyridine infrared spectrum of the Ni-SCM-14 catalyst in Example 1;
FIG. 4 shows an XPS spectrum of the Ni-SCM-14 catalyst in Example 1;
FIG. 5A shows a TEM image and FIG. 5B shows a particle size distribution diagram of the Ni-SCM-14 catalyst in Example 1;
FIG. 6 shows an XRD pattern of the Ni-SCM-15 catalyst in Example 4; and
FIG. 7A shows a TEM image and FIG. 7B shows a particle size distribution diagram of the Ni-SCM-14 catalyst in Comparative Example 1.

## DETAILED DESCRIPTION

In a first aspect, the present disclosure provides a Ni-SCM-X molecular sieve catalyst, wherein X is 14 or 15; Ni comprises metallic Ni and $Ni^{2+}$, wherein the molar ratio of $Ni/Ni^{2+}$ is in a range of 0.02-0.15, preferably 0.05-0.12.

In some embodiments, in the catalyst, the molar ratio of metallic Ni to $Ni^{2+}$ ($Ni/Ni^{2+}$) may be 0.05, 0.06, 0.07, 0.08,

0.09, 0.10, 0.11, 0.12, 0.13, or in a range between any two of the above values.

The catalyst of the present disclosure is a solid catalyst with Ni supported on an SCM-X molecular sieve, wherein X is 14 or 15. In the present disclosure, Ni comprises metallic Ni and $Ni^{2+}$, wherein metallic Ni means that Ni exists in metallic (zero-valent) form. In some embodiments, $Ni^{2+}$ exists in an oxidized state (NiO).

In the present disclosure, the catalyst comprises nickel particles. In some embodiments, the nickel in the catalyst is present as particles. In some embodiments, the mean particle size of the nickel particles in the catalyst is in a range of 2.0-8.0 nm, preferably 3.0-7.0 nm, preferably 3.0-6.5 nm, more preferably 3.0-6.0 nm.

In some embodiments, in the catalyst, based on the mass of the SCM-X molecular sieve, the content of Ni, calculated as elemental Ni, is in a range of 0.2 wt%-5.0 wt%, preferably 0.5 wt%-3.0 wt%. For example, based on the mass of the SCM-X molecular sieve, the content of Ni, calculated as elemental Ni, may be 0.5 wt%, 0.6 wt%, 0.7 wt%, 0.8 wt%, 0.9 wt%, 1 wt%, 1.5 wt%, 2 wt%, 2.5 wt%, 3 wt%, or in a range between any two of the above values.

In the present disclosure, in the SCM-X molecular sieve, X is 14 or 15; that is, the SCM-X molecular sieve is an SCM-14 molecular sieve, an SCM-15 molecular sieve, or a combination of an SCM-14 molecular sieve and an SCM-15 molecular sieve. The SCM-14 molecular sieve and the preparation method thereof are described in CN109081360A and WO2018227849A1, the entire contents of which are incorporated herein by reference. The SCM-15 molecular sieve and the preparation method thereof are described in CN109081359A and WO2018227850A1, the entire contents of which are incorporated herein by reference. In the present disclosure, the SCM-X molecular sieve, wherein X is 14 or 15, can be prepared by the methods described in CN109081360A/WO2018227849A1 and CN109081359A/WO2018227850A1, respectively.

In some embodiments, in the Ni-SCM-X molecular sieve catalyst, the Si/Ge molar ratio of the SCM-X molecular sieve (after calcination) is in a range of 1.5-8, preferably 2-7. For example, the Si/Ge molar ratio may be 2, 3, 4, 5, 6, or 7, or in a range between any two of the above values. In the present disclosure, inductively coupled plasma atomic emission spectrometry can be used to measure the content of metal elements. The sample can be dissolved with hydrofluoric acid and then tested to obtain the content of metal elements.

In some embodiments, the total specific surface area of the catalyst is in a range of 90-370 $m^2 \cdot g^{-1}$, preferably 100-350 $m^2 \cdot g^{-1}$. In some embodiments, the total specific surface area of the catalyst may be 100 $m^2 \cdot g^{-1}$, 110 $m^2 \cdot g^{-1}$, 120 $m^2 \cdot g^{-1}$, 130 $m^2 \cdot g^{-1}$, 140 $m^2 \cdot g^{-1}$, 150 $m^2 \cdot g^{-1}$, 160 $m^2 \cdot g^{-1}$, 200 $m^2 \cdot g^{-1}$, 250 $m^2 \cdot g^{-1}$, 300 $m^2 \cdot g^{-1}$, or in a range between any two of the above values. In the present disclosure, the specific surface area can be measured by the BET method.

In some embodiments, the total acid amount of the catalyst is in a range of 130-220 $\mu mol \cdot g^{-1}$, preferably 150-200 $\mu mol \cdot g^{-1}$. In some embodiments, the total acid amount of the catalyst is 150 $\mu mol \cdot g^{-1}$, 160 $\mu mol \cdot g^{-1}$, 170 $\mu mol \cdot g^{-1}$, 180 $\mu mol \cdot g^{-1}$, 190 $\mu mol \cdot g^{-1}$, 200 $\mu mol \cdot g^{-1}$, or in a range between any two of the above values. In the present disclosure, the total acid amount is the total acid amount determined by $NH_3$ temperature-programmed desorption ($NH_3$-TPD) experiment.

In some embodiments, the Brønsted acid/Lewis acid ratio of the catalyst is in a range of 0.3-0.8, preferably 0.35-0.6, more preferably 0.4-0.6. In the present disclosure, the acid type and acid amount can be determined by pyridine adsorption infrared method, and the Brønsted acid/Lewis acid ratio can be calculated.

The catalyst of the present disclosure can be used for catalyzing hydrogenation reactions. In some embodiments, the catalyst of the present disclosure can be used for catalyzing the conversion of 5-hydroxymethylfurfural (HMF) and/or 5-(C1-C20 alkoxymethyl)furfural to prepare 2,5-dimethylfuran, for example, in the presence of hydrogen. In some embodiments, the catalyst of the present disclosure can be used for catalyzing the conversion of hydroxybenzaldehyde to prepare methylphenol, for example, in the presence of hydrogen. The catalyst of the present disclosure can provide high conversion and selectivity in these reactions, and has excellent stability.

In the present disclosure, 5-(C1-C20 alkoxymethyl)furfural is a compound in which the 5-hydroxymethyl group of 5-hydroxymethylfurfural is converted to a 5-(C1-C20 alkoxymethyl) group. Preferably, the 5-(C1-C20 alkoxymethyl) furfural is 5-(C1-C8 alkoxymethyl)furfural, more preferably 5-(C1-C5 alkoxymethyl)furfural. In the present disclosure, the C1-C5 alkoxy group may be methoxy, ethoxy, n-propoxy, isopropoxy, n-butoxy, isobutoxy, tert-butoxy, isoamyloxy, etc.

In a second aspect, the present disclosure provides a method for preparing the above catalyst, comprising: mixing an SCM-X molecular sieve with a nickel-containing precursor to obtain a mixture, and calcining and reducing the obtained mixture to obtain the Ni-SCM-X molecular sieve catalyst, wherein X is 14 or 15.

In some embodiments, in the preparation method, before calcining and reducing the mixture, the mixture is subjected to drying.

In some embodiments, the nickel-containing precursor can be selected from nickel precursors containing an organic ligand (organometallic precursors). In some embodiments, preferably, the organic ligand is selected from organic ligands containing a five-membered carbocyclic ring, a six-membered carbocyclic ring, or an eight-membered carbocyclic ring. In some embodiments, preferably the five-membered carbocyclic ring may be a cyclopentadiene ring, the six-membered carbocyclic ring may be a benzene ring, and the eight-membered carbocyclic ring may be a cyclooctadiene ring. The five-membered carbocyclic ring, six-membered carbocyclic ring or eight-membered carbocyclic ring optionally have substituent(s), for example C1-C4 alkyl group. In some embodiments, preferably the nickel-containing precursor is

selected from at least one of nickelocene (bis(cyclopentadienyl) nickel), bis-(1,5-cyclooctadiene) nickel, bis(tetramethylcyclopentadienyl) nickel, 1,2-bis(diphenylphosphino)ethane nickel chloride and bis(triphenylphosphine) nickel chloride, and further more preferably nickelocene.

In some embodiments, in the mixing step, the mass ratio of Ni (the amount of Ni in the nickel-containing precursor) to the SCM-X molecular sieve is in a range of 0.2-8.0:100, preferably 0.5-5.0:100. In some embodiments, for example, the mass ratio of Ni (the amount of Ni in the nickel-containing precursor) to the SCM-X molecular sieve may be 0.6:100, 0.7:100, 0.8:100, 0.9:100, 1:100, 1.5:100, 2:100, 2.5:100, 3:100, 3.5:100, 4:100, 4.5:100, or in a range between any two of the above values.

In the method of the present disclosure, preferably, the mixing is physical mixing. In some embodiments, the mixing includes but is not limited to grinding, ball milling, etc. For example, the grinding can be carried out in a mortar. For example, the ball milling can be carried out in a ball mill. A person skilled in the art can appropriately determine the mixing time as long as uniform mixing can be achieved.

In the method of the present disclosure, after mixing the SCM-X molecular sieve with the nickel-containing precursor, a uniform mixture is obtained. After mixing, optionally, the obtained mixture can be dried. The drying temperature may be in a range of 50-130°C, for example, may be 60°C, 70°C, 80°C, 90°C, 100°C, 110°C, 120°C, 130°C, or in a range between any two of the above values. The drying time may be in a range of 2-8 h, for example, may be 3 h, 4 h, 5 h, 6 h, 7 h, 8 h, or in a range between any two of the above values. In some embodiments, the conditions for drying may include drying at 50-130°C for 2-8 h. The drying can be carried out, for example, in an air atmosphere.

In the method of the present disclosure, after optional drying, the dried product is subjected to a calcination. The calcination temperature may be in a range of 300-650°C, for example, may be 350°C, 400°C, 450°C, 500°C, 550°C, 600°C, 620°C, 650°C, or in a range between any two of the above values. The calcination time may be in a range of 1-12 h, for example, may be 2 h, 3 h, 4 h, 5 h, 6 h, 7 h, 8 h, 9 h, 10 h, 11 h, 12 h, or in a range between any two of the above values. The calcination atmosphere may be an oxygen-containing gas, preferably oxygen, air, or a mixed gas of oxygen and air. In some embodiments, the conditions for the calcination may include: calcining at 300-650°C for 1-12 h, and the calcination atmosphere is an oxygen-containing gas, preferably oxygen, air, or a mixed gas of oxygen and air. When a mixed gas of oxygen and air is used, oxygen and air may be mixed in any proportion.

In the method of the present disclosure, after calcining, the calcined product is subjected to a reduction treatment. In some embodiments, the reduction is carried out under a hydrogen atmosphere. The reduction temperature may be in a range of 300-500°C, preferably 350-450°C, for example, may be 360°C, 370°C, 380°C, 390°C, 400°C, 410°C, 420°C, 430°C, 440°C, 450°C, or in a range between any two of the above values. The reduction time may be in a range of 1-8 h, for example, may be 2 h, 3 h, 4 h, 5 h, 6 h, 7 h, 8 h, or in a range between any two of the above values. In some embodiments, the reduction temperature may be in a range of 300-500°C, preferably 350-450°C; and the reduction time may be in a range of 1-8 h, preferably 2-6 h. In some embodiments, the reduction is carried out under a hydrogen atmosphere at atmospheric pressure.

In a third aspect, the present disclosure provides a method for preparing 2,5-dimethylfuran, comprising:
in the presence of the catalyst according to the present disclosure, subjecting 5-hydroxymethylfurfural and/or 5-(C1-C20 alkoxymethyl)furfural to hydrogenolysis in an organic solvent to prepare 2,5-dimethylfuran.

The method of the present disclosure has no particular limitation on the conditions for the hydrogenolysis reaction of 5-hydroxymethylfurfural, as long as 2,5-dimethylfuran can be prepared. In some embodiments, the hydrogenolysis is carried out by using hydrogen. The reaction temperature of the hydrogenolysis reaction may be in a range of 130-220°C, for example, may be 140°C, 150°C, 160°C, 170°C, 180°C, 190°C, 200°C, or in a range between any two of the above values. The reaction time of the hydrogenolysis reaction may be in a range of 1-12 h, for example, may be 2 h, 3 h, 4 h, 5 h, 6 h, 7 h, 8 h, 9 h, 10 h, 11 h, or in a range between any two of the above values. The hydrogen pressure may be in a range of 0.5-4 MPa, for example, may be 0.6 MPa, 0.8 MPa, 1 MPa, 1.5 MPa, 2 MPa, 2.5 MPa, 3 MPa, 3.5 MPa, or in a range between any two of the above values. In some embodiments, preferably, the conditions for the hydrogenolysis reaction may include: a reaction temperature in a range of 130-220°C, preferably a reaction temperature in a range of 150-200°C; a reaction time in a range of 1-12 h, preferably a reaction time in a range of 2-10 h; and a hydrogen pressure in a range of 0.5-4 MPa, preferably a hydrogen pressure in a range of 1-3 MPa.

The method of the present disclosure has no particular limitation on the organic solvent, as long as 2,5-dimethylfuran can be prepared. Solvents commonly known in the art for preparing 2,5-dimethylfuran can be used. In some embodiments of the present disclosure, the organic solvent may comprise at least one of C1-C5 alcohols, cyclic ether compounds, γ-valerolactone, methyl isobutyl ketone, and acetone, preferably the C1-C5 alcohol is selected from methanol, ethanol, n-propanol, n-butanol, isobutanol, isopropanol, isoamyl alcohol, ethylene glycol, propylene glycol and glycerol, and the cyclic ether compound is selected from pyran, 2-methyltetrahydrofuran, 2,5-dimethyltetrahydrofuran, tetrahydrofuran and 1,4-dioxane. In some embodiments, preferably, the organic solvent may comprise at least one of tetrahydrofuran, n-butanol, γ-valerolactone, methyl isobutyl ketone, and acetone, more preferably comprises tetrahydrofuran.

In some embodiments, the organic solvent comprises a mixture of solvent I and solvent II; the solvent I is a C1-C5

alcohol solvent, preferably at least one of methanol, ethanol, n-propanol, n-butanol, isobutanol, isopropanol, isoamyl alcohol, ethylene glycol, propylene glycol or glycerol, further preferably one or two of isopropanol, isoamyl alcohol or n-butanol; and the solvent II is selected from at least one of cyclic ether compounds, acetone, methyl isobutyl ketone, and $\gamma$-valerolactone, wherein the cyclic ether compound is preferably at least one of pyran, 2-methyltetrahydrofuran, 2,5-dimethyltetrahydrofuran, tetrahydrofuran and 1,4-dioxane, and the solvent II is preferably one or two of acetone, 1,4-dioxane and tetrahydrofuran. In some embodiments, preferably the mass ratio of solvent I to solvent II may be in a range of 0.1-15:1, more preferably 0.5-10:1.

In the present disclosure, the reactant (also referred to as substrate) used for preparing 2,5-dimethylfuran is selected from 5-hydroxymethylfurfural, 5-(C1-C20 alkoxymethyl)furfural, and any mixture thereof. In some embodiments, the reactant may comprise 5-hydroxymethylfurfural and 5-(C1-C20 alkoxymethyl)furfural, preferably the molar ratio of 5-hydroxymethylfurfural to 5-(C1-C20 alkoxymethyl)furfural may be in a range of 0.5-1.5. Preferably, the 5-(C1-C20 alkoxymethyl)furfural is 5-(C1-C8 alkoxymethyl)furfural, more preferably 5-(C1-C5 alkoxymethyl)furfural.

In the present disclosure, a person skilled in the art can appropriately select the amounts of the organic solvent, catalyst, and reactant (substrate). In some embodiments, the mass ratio of 5-hydroxymethylfurfural and/or 5-(C1-C20 alkoxymethyl)furfural (substrate) to the catalyst may be in a range of 0.2-5:1, preferably 0.5-4:1, for example, may be 0.6:1, 0.7:1, 0.8:1, 0.9:1, 1:1, 1.5:1, 2:1, 2.5:1, 3:1, 3.5:1, or in a range between any two of the above values. In some embodiments, the mass ratio of the organic solvent to 5-hydroxymethylfurfural and/or 5-(C1-C20 alkoxymethyl)furfural (substrate) may be in a range of 40-400:1, preferably 50-300:1, for example, may be 60:1, 70:1, 80:1, 90:1, 100:1, 150:1, 180:1, 200:1, 250:1, or in a range between any two of the above values.

The present disclosure also provides use of the catalyst according to the present disclosure or the catalyst prepared by the preparation method according to the present disclosure as a catalyst in a reaction for preparing 2,5-dimethylfuran from 5-hydroxymethylfurfural and/or 5-(C1-C20 alkoxymethyl)furfural.

In the present disclosure, pressure refers to gauge pressure, unless otherwise specified.

The present disclosure can provide the following beneficial effects:

In the present disclosure, the catalyst has uniform Ni distribution, small particle size of the particles, and a suitable molar ratio of metallic Ni to $Ni^{2+}$. When used in the reaction for catalyzing the conversion of 5-hydroxymethylfurfural and/or 5-(C1-C20 alkoxymethyl)furfural to prepare 2,5-dimethylfuran, it has the advantages of high conversion of 5-hydroxymethylfurfural and/or 5-(C1-C20 alkoxymethyl)furfural and high selectivity for the product 2,5-dimethylfuran under mild reaction conditions, as well as high stability of the catalyst upon recycling. In the method of the present disclosure, the selectivity for the by-product 5-methylfurfural is very low, preferably the selectivity for 5-methylfurfural is less than 2%, for example less than 1.5%, more preferably less than 1%, even more preferably less than 0.8%.

In the present disclosure, the Ni-SCM-X molecular sieve catalyst is obtained by mixing an SCM-X molecular sieve with a nickel source containing an organic ligand, and drying (optional), calcining and reducing the obtained mixture. Wherein, the organic ligand has a relatively large size and a strong interaction force with nickel, so that the nickel is uniformly distributed, the nickel particles have a uniform particle size and a small particle size. In addition, the catalyst of the present disclosure has a suitable molar ratio of metallic Ni to $Ni^{2+}$. In the present disclosure, under mild reaction conditions, 5-hydroxymethylfurfural and/or 5-(C1-C20 alkoxymethyl)furfural can be efficiently converted to 2,5-dimethylfuran, and both the substrate conversion and the product selectivity are significantly improved. In addition, the prepared Ni-SCM-X molecular sieve catalyst has good stability, for example, no significant change is observed after four cycles of use.

In the present disclosure, the reaction product 2,5-dimethylfuran (DMF) was qualitatively analyzed by gas chromatography-mass spectrometry (GC-MS), and the substrate conversion and the yield of the reaction product DMF were analyzed by gas chromatography (GC). The gas chromatography-mass spectrometer was an Agilent (USA) G7077BA\MSD, with an INNOWAX gas chromatography capillary column (60 m, 0.32 mm); the gas chromatograph was an Agilent 8860, with a flame ionization detector (FID), and an INNOWAX gas chromatography capillary column (60 m, 0.32 mm).

In the present disclosure, the XRD measurement method included: using a Rigaku (Japan) Ultima IV X-ray powder diffractometer, CuK$\alpha$ radiation source ($\lambda$ = 1.54 Å), nickel filter, 2$\theta$ scanning range 2°-50°/75°, operating voltage 35 kV, current 25 mA, scanning rate 10°/min.

In the present disclosure, the model of the inductively coupled plasma atomic emission spectrometer (ICP) used was Varian 725-ES. The sample was dissolved with hydrofluoric acid to detect the content of metal elements.

In the present disclosure, $NH_3$ temperature-programmed desorption ($NH_3$-TPD) experiments were carried out on a TPD/TPR Altamira AMI-3300 instrument, and the obtained spectra were fitted and peak-separated to calculate the total acid amount.

The acid type and acid amount of the catalyst were determined by pyridine adsorption infrared method. The instrument used was a Nicolet Model 710 spectrometer. The specific operation steps were as follows: a. Sample pretreatment: the sample (about 30 mg) was pressed into a thin circular disk with a diameter of 13 mm and placed in an infrared sample cell; then, the sample was pretreated at 400°C for 1 h under vacuum cell conditions; after the sample cell was cooled to room temperature, the infrared data of the sample was scanned as background; b. Pyridine adsorption:

at room temperature and under vacuum environment, pyridine vapor was introduced into the in-situ cell until adsorption reached equilibrium, the adsorption time was 1 h, and then the infrared absorption spectrum was scanned and recorded; c. Pyridine desorption: after adsorption, vacuum was applied at 100°C until the internal pressure no longer changed (about $10^{-3}$ Pa), the desorption time was 40 min, and then the infrared absorption spectrum was scanned and recorded. The difference spectrum before and after pyridine adsorption was the obtained pyridine adsorption-infrared absorption spectrum. According to the spectrum, the acid amount of the sample was calculated using the following formulas:

$$C_L = K_L \times A_{1440} = \frac{\pi}{IMEC_L} \times \left(\frac{r^2}{w}\right) \times A_{1440}$$

$$C_B = K_B \times A_{1540} = \frac{\pi}{IMEC_B} \times \left(\frac{r^2}{w}\right) \times A_{1540}$$

wherein, r and w are the diameter (cm) and mass (g) of the thin circular disk of the catalyst, respectively, and A is the integrated absorbance value of the peak at the specified wavenumber according to the scanned pyridine adsorption-infrared absorption spectrum. IMEC is the integrated molar extinction coefficient, wherein $IMEC_L$ is 2.22 and $IMEC_B$ is 1.67.

In the present disclosure, the model of the transmission electron microscope (TEM) used was JEOL JEM-2100F electron microscope (200 kV). The particle size of nickel particles in the molecular sieve catalyst was obtained by randomly selecting about 30 nickel particles from the TEM image for particle size statistics, and then calculating the arithmetic mean value.

In the present disclosure, the determination of the surface element binding energy of the catalyst was carried out on a Thermo ESCA LAB-250 X-ray photoelectron spectrometer, using C1s = 284.6 eV as an internal standard to calibrate the measured element signals.

In the present disclosure, the specific surface area of the catalyst was measured by the nitrogen physical adsorption-desorption method (BET method). The nitrogen physical adsorption-desorption isotherms of the molecular sieve were measured using a physical adsorption instrument (Micromeritics ASAP 2020M). The obtained results were analyzed by the Brunauer-Emmet-Teller (BET) method to obtain the specific surface area of the sample, and by the Barrett-Joyner-Halenda (BJH) method to obtain the pore volume and pore size distribution of the sample. Experimental conditions: the sample was vacuum degassed at 350°C for 4 h before analysis to remove moisture and volatile impurities, and then the nitrogen adsorption-desorption test was performed in a liquid nitrogen environment (-196°C).

In the present disclosure, the calculation formula for the conversion of the substrate (5-hydroxymethylfurfural and/or 5-(C1-C20 alkoxymethyl)furfural) is:

Substrate conversion % = (moles of substrate reacted) / (moles of substrate) × 100%.

In the present disclosure, the calculation formula for the yield of the product DMF is:

Product DMF yield % = (moles of DMF produced by the reaction) / (moles of substrate) × 100%.

In the present disclosure, the calculation formula for the selectivity of the product DMF is:

Product DMF selectivity % = (moles of DMF produced by the reaction) / (moles of substrate reacted) × 100%.

In the present disclosure, the calculation formula for the selectivity of the by-product 5-methylfurfural is:

5-Methylfurfural selectivity % = (moles of 5-methylfurfural produced by the reaction) / (moles of substrate reacted) × 100%.

To facilitate understanding of the present disclosure, the present disclosure provides examples as follows. However, these examples are only for helping to understand the present disclosure and should not be considered as specific limitations on the present disclosure.

In the following examples, unless otherwise specified, all reagents used were commercially available.

**Example 1**

An SCM-14 molecular sieve was synthesized according to the preparation method described in Example 1 of CN109081360A, wherein the Si/Ge molar ratio was 3.7. The nickel-containing precursor was nickelocene. The mass ratio of Ni (the amount of Ni in the organometallic precursor) to the above SCM-14 molecular sieve was 1.0:100. The amount of SCM-14 added was 3.0 g. The mixture was thoroughly ground in a mortar to obtain a uniformly dispersed mixture. The above product was dried in an oven at 100°C for 4 h, then calcined at 550°C for 4 h, and reduced under atmospheric hydrogen conditions at a reduction temperature of 400°C for 3 h, to obtain a Ni-SCM-14 molecular sieve catalyst.

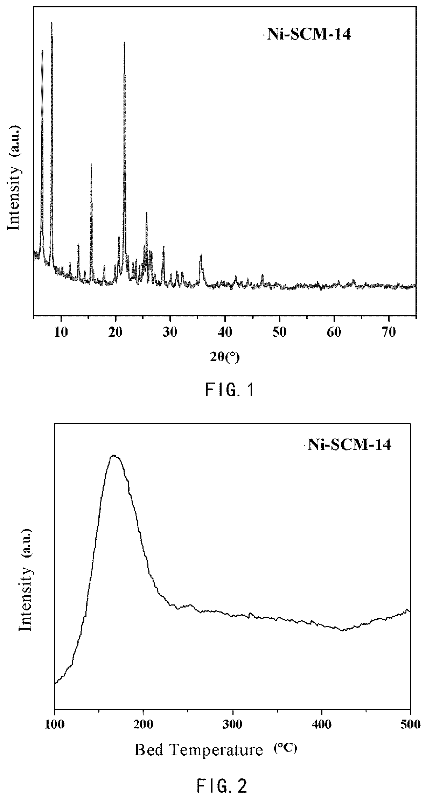

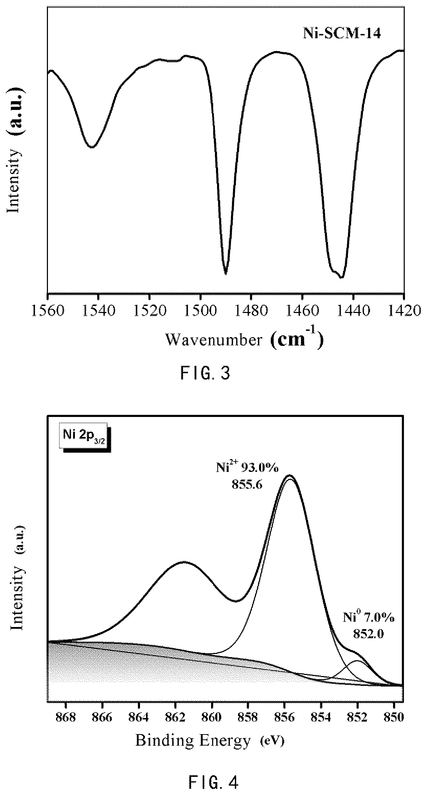

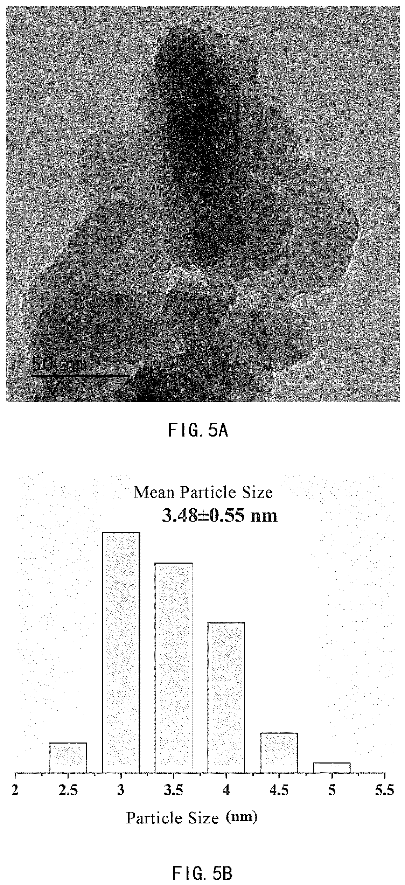

The Si/Ge molar ratio of the sample measured by the inductively coupled plasma atomic emission spectrometer (ICP) was 3.7, and the relative content of Ni was 1.0 wt%. The specific surface area of the sample measured by the BET method was 138 $m^2 \cdot g^{-1}$. The XRD pattern of the sample is shown in FIG. 1. The $NH_3$-TPD pattern of the sample is shown in FIG. 2, and the total acid amount calculated therefrom was 175 $\mu mol \cdot g^{-1}$. The pyridine infrared spectrum of the sample is shown in FIG. 3, and the Brønsted acid/Lewis acid ratio calculated therefrom was 0.50. The XPS spectrum of the sample is shown in FIG. 4. The peak at a binding energy of 852.0 eV corresponds to metallic Ni ($2p_{3/2}$ peak), and the peak at a binding energy of 855.6 eV corresponds to divalent nickel ($2^+$). The molar ratio of metallic Ni to $Ni^{2+}$ ($Ni/Ni^{2+}$ ratio) calculated from the ratio of the two peak areas was 0.08. The TEM image of the sample is shown in FIG. 5A, showing that the distribution of nickel was uniform without obvious aggregation, and the mean particle size of nickel particles was 3.5 nm.

**Example 2**

An SCM-14 molecular sieve was synthesized according to the preparation method described in Example 1 of CN109081360A, wherein the Si/Ge molar ratio was 3.7. The nickel-containing precursor was nickelocene. The mass ratio of Ni (the amount of Ni in the organometallic precursor) to the above SCM-14 molecular sieve was 2.0:100. The amount of SCM-14 added was 3.0 g. The mixture was thoroughly ground in a mortar to obtain a uniformly dispersed mixture. The above product was dried in an oven at 80°C for 4 h, then calcined at 550°C for 4 h, and reduced under atmospheric hydrogen conditions at a reduction temperature of 380°C for 4 h, to obtain a Ni-SCM-14 molecular sieve catalyst.

The Si/Ge molar ratio of the sample measured by the inductively coupled plasma atomic emission spectrometer (ICP) was 3.7, and the relative content of Ni was 2.0 wt%. The specific surface area of the sample measured by the BET method was 135 $m^2 \cdot g^{-1}$. The XRD pattern of the sample was similar to FIG. 1. The $NH_3$-TPD pattern of the sample was similar to FIG. 2, and the total acid amount calculated therefrom was 183 $\mu mol \cdot g^{-1}$. The pyridine infrared spectrum of the sample was similar to FIG. 3, and the Brønsted acid/Lewis acid ratio calculated therefrom was 0.57. The XPS spectrum of the sample was similar to FIG. 4. The peak at a binding energy of 852.0 eV corresponds to metallic Ni ($2p_{3/2}$ peak), and the peak at a binding energy of 855.6 eV corresponds to divalent nickel ($2^+$). The molar ratio of metallic Ni to $Ni^{2+}$ ($Ni/Ni^{2+}$ ratio) calculated from the ratio of the two peak areas was 0.09. The TEM image of the sample was similar to FIG. 5A, showing that the distribution of nickel was uniform without obvious aggregation, and the mean particle size of nickel particles was 3.3 nm.

**Example 3**

An SCM-14 molecular sieve was synthesized according to the preparation method described in Example 2 of CN109081360A, wherein the Si/Ge molar ratio was 3.5. The nickel-containing precursor was nickelocene. The mass ratio of Ni (the amount of Ni in the organometallic precursor) to the above SCM-14 molecular sieve was 1.5:100. The amount of SCM-14 added was 3.0 g. The mixture was thoroughly ground in a mortar to obtain a uniformly dispersed mixture. The above product was dried in an oven at 90°C for 4 h, then calcined at 550°C for 4 h, and reduced under atmospheric hydrogen conditions at a reduction temperature of 410°C for 3 h, to obtain a Ni-SCM-14 molecular sieve catalyst.

The Si/Ge molar ratio of the sample measured by the inductively coupled plasma atomic emission spectrometer (ICP) was 3.5, and the relative content of Ni was 1.5 wt%. The specific surface area of the sample measured by the BET method was 131 $m^2 \cdot g^{-1}$. The XRD pattern of the sample was similar to FIG. 1. The $NH_3$-TPD pattern of the sample was similar to FIG. 2, and the total acid amount calculated therefrom was 172 $\mu mol \cdot g^{-1}$. The pyridine infrared spectrum of the sample was similar to FIG. 3, and the Brønsted acid/Lewis acid ratio calculated therefrom was 0.52. The XPS spectrum of the sample was similar to FIG. 4. The peak at a binding energy of 852.0 eV corresponds to metallic Ni ($2p_{3/2}$ peak), and the peak at a binding energy of 855.6 eV corresponds to divalent nickel ($2^+$). The molar ratio of metallic Ni to $Ni^{2+}$ ($Ni/Ni^{2+}$ ratio) calculated from the ratio of the two peak areas was 0.07. The TEM image of the sample was similar to FIG. 5A, showing that the distribution of nickel was uniform without obvious aggregation, and the mean particle size of nickel particles was 3.7 nm.

**Example 4**

An SCM-15 molecular sieve was synthesized according to the preparation method described in Example 1 of CN109081359A, wherein the Si/Ge molar ratio was 5.1. The nickel-containing precursor was nickelocene. The mass ratio of Ni (the amount of Ni in the organometallic precursor) to the above SCM-15 molecular sieve was 1.0:100. The amount of SCM-15 added was 3.0 g. The mixture was thoroughly ground in a mortar to obtain a uniformly dispersed mixture. The above product was dried in an oven at 80°C for 5 h, then calcined at 550°C for 4 h, and reduced under atmospheric hydrogen conditions at a reduction temperature of 400°C for 2 h, to obtain a Ni-SCM-15 molecular sieve catalyst.

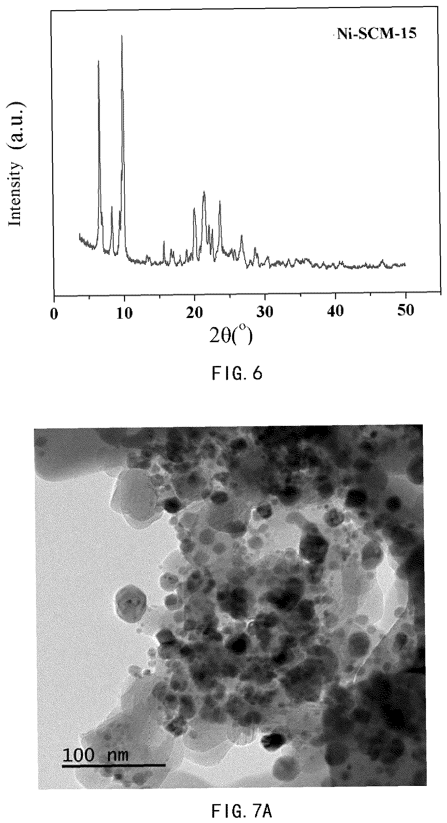

The Si/Ge molar ratio of the sample measured by the inductively coupled plasma atomic emission spectrometer (ICP) was 5.1, and the relative content of Ni was 1.0 wt%. The specific surface area of the sample measured by the BET method was 325 $m^2 \cdot g^{-1}$. The XRD pattern of the sample is shown in FIG. 6. The $NH_3$-TPD pattern of the sample was similar to FIG. 2, and the total acid amount calculated therefrom was 157 $\mu mol \cdot g^{-1}$. The pyridine infrared spectrum of the sample was similar to FIG. 3, and the Brønsted acid/Lewis acid ratio calculated therefrom was 0.46. The XPS spectrum of the sample was similar to FIG. 4. The peak at a binding energy of 852.0 eV corresponds to metallic Ni ($2p_{3/2}$ peak), and the peak at a binding energy of 855.6 eV corresponds to divalent nickel ($2^+$). The molar ratio of metallic Ni to $Ni^{2+}$ ($Ni/Ni^{2+}$ ratio) calculated from the ratio of the two peak areas was 0.10. The TEM image of the sample was similar to FIG. 5A, showing that the distribution of nickel was uniform without obvious aggregation, and the mean particle size of nickel particles was 4.1 nm.

**Example 5**

An SCM-14 molecular sieve was synthesized according to the preparation method described in Example 1 of CN109081360A, wherein the Si/Ge molar ratio was 3.7. The nickel-containing precursor was bis-(1,5-cyclooctadiene) nickel. The mass ratio of Ni (the amount of Ni in the organometallic precursor) to the above SCM-14 molecular sieve was 0.9:100. The amount of SCM-14 added was 3.0 g. The mixture was thoroughly ground in a mortar to obtain a uniformly dispersed mixture. The above product was dried in an oven at 100°C for 4 h, then calcined at 550°C for 4 h, and reduced under atmospheric hydrogen conditions at a reduction temperature of 400°C for 3 h, to obtain a Ni-SCM-14 molecular sieve catalyst.

The Si/Ge molar ratio of the sample measured by the inductively coupled plasma atomic emission spectrometer (ICP) was 3.7, and the relative content of Ni was 0.9 wt%. The specific surface area of the sample measured by the BET method was 136 $m^2 \cdot g^{-1}$. The XRD pattern of the sample was similar to FIG. 1. The $NH_3$-TPD pattern of the sample was similar to FIG. 2, and the total acid amount calculated therefrom was 143 $\mu mol \cdot g^{-1}$. The pyridine infrared spectrum of the sample was similar to FIG. 3, and the Brønsted acid/Lewis acid ratio calculated therefrom was 0.47. The XPS spectrum of the sample was similar to FIG. 4. The peak at a binding energy of 852.0 eV corresponds to metallic Ni ($2p_{3/2}$ peak), and the peak at a binding energy of 855.6 eV corresponds to divalent nickel ($2^+$). The molar ratio of metallic Ni to $Ni^{2+}$ ($Ni/Ni^{2+}$ ratio) calculated from the ratio of the two peak areas was 0.13. The TEM image of the sample was similar to FIG. 5A, and the mean particle size of nickel particles was 5.5 nm.

**Example 6**

An SCM-14 molecular sieve was synthesized according to the preparation method described in Example 2 of CN109081360A, wherein the Si/Ge molar ratio was 3.5. The nickel-containing precursor was bis(tetramethylcyclopentadienyl) nickel. The mass ratio of Ni (the amount of Ni in the organometallic precursor) to the above SCM-14 molecular sieve was 1.3:100. The amount of SCM-14 added was 3.0 g. The mixture was thoroughly ground in a mortar to obtain a uniformly dispersed mixture. The above product was dried in an oven at 100°C for 4 h, then calcined at 550°C for 4 h, and reduced under atmospheric hydrogen conditions at a reduction temperature of 400°C for 3 h, to obtain a Ni-SCM-14 molecular sieve catalyst.

The Si/Ge molar ratio of the sample measured by the inductively coupled plasma atomic emission spectrometer (ICP) was 3.5, and the relative content of Ni was 1.3 wt%. The specific surface area of the sample measured by the BET method was 142 $m^2 \cdot g^{-1}$. The XRD pattern of the sample was similar to FIG. 1. The $NH_3$-TPD pattern of the sample was similar to FIG. 2, and the total acid amount calculated therefrom was 161 $\mu mol \cdot g^{-1}$. The pyridine infrared spectrum of the sample was similar to FIG. 3, and the Brønsted acid/Lewis acid ratio calculated therefrom was 0.36. The XPS spectrum of the sample was similar to FIG. 4. The peak at a binding energy of 852.0 eV corresponds to metallic Ni ($2p_{3/2}$ peak), and the peak at a binding energy of 855.6 eV corresponds to divalent nickel ($2^+$). The molar ratio of metallic Ni to $Ni^{2+}$ ($Ni/Ni^{2+}$ ratio) calculated from the ratio of the two peak areas was 0.11. The TEM image of the sample was similar to FIG. 5A, and the mean particle size of nickel particles was 5.8 nm.

## Examples 7-12

The catalysts in accordance with the present disclosure were used in the reaction for converting HMF to DMF. Tetrahydrofuran was used as the reaction solvent, the mass ratio of tetrahydrofuran to HMF was 100, the mass ratio of HMF to catalyst was 1, the hydrogen pressure was 1.5 MPa, the reaction temperature was 190°C, and the reaction time was 3 h.

0.2 g of the catalyst from each of Examples 1-6 above, 0.2 g of HMF, and 20 g of tetrahydrofuran were added to an autoclave with stirrer, and 1.5 MPa of hydrogen was charged. The temperature was raised to the preset temperature using a programmed heating mantle, and stirring was performed with a magnetic stirrer. The reaction was carried out at 190°C for 3 h. The reaction liquid was analyzed by gas chromatography/gas chromatography-mass spectrometry to calculate the HMF conversion and the selectivities for the product DMF and the key by-product 5-methylfurfural, which are shown in Table 1.

Table 1. Catalytic evaluation results of the catalysts in Examples 1-6

| Ex. No. | Catalyst | HMF Conversion (%) | DMF Selectivity (%) | 5-Methylfurfural Selectivity (%) |
|---|---|---|---|---|
| 7 | Example 1 | >99 | 90.1% | 0.2 |
| 8 | Example 2 | >99 | 89.2% | 0.1 |
| 9 | Example 3 | >99 | 88.5% | 0.3 |
| 10 | Example 4 | >99 | 87.7% | 0.2 |
| 11 | Example 5 | >99 | 82.6% | 0.5 |
| 12 | Example 6 | >99 | 83.2% | 0.6 |

## Example 13

Tetrahydrofuran was used as the reaction solvent, the mass ratio of tetrahydrofuran to HMF was 80, the mass ratio of HMF to catalyst was 2, the hydrogen pressure was 2.5 MPa, the reaction temperature was 170°C, and the reaction time was 6 h.

0.2 g of the Ni-SCM-14 catalyst from Example 1 above, 0.4 g of HMF, and 32 g of tetrahydrofuran were added to an autoclave with stirrer, and 2.5 MPa of hydrogen was charged. The temperature was raised to the preset temperature using a programmed heating mantle, and stirring was performed with a magnetic stirrer. The reaction was carried out at 190°C for 3 h. The reaction liquid was analyzed by gas chromatography/gas chromatography-mass spectrometry to calculate the conversion and selectivities. The HMF conversion was >99%, the selectivity for the product DMF was 87.5%, and the selectivity for the key by-product 5-methylfurfural was 0.3%.

## Example 14

Tetrahydrofuran was used as the reaction solvent, the mass ratio of tetrahydrofuran to HMF was 80, the mass ratio of HMF to catalyst was 0.8, the hydrogen pressure was 1 MPa, the reaction temperature was 190°C, and the reaction time was 2 h.

0.2 g of the Ni-SCM-14 catalyst from Example 1 above, 0.16 g of HMF, and 12.8 g of tetrahydrofuran were added to an autoclave with stirrer, and 1 MPa of hydrogen was charged. The temperature was raised to the preset temperature using a programmed heating mantle, and stirring was performed with a magnetic stirrer. The reaction was carried out at 190°C for 2 h. The reaction liquid was analyzed by gas chromatography/gas chromatography-mass spectrometry to calculate the conversion and selectivities. The HMF conversion was >99%, the selectivity for the product DMF was 89.1%, and the selectivity for the key by-product 5-methylfurfural was 0.1%.

## Example 15

Tetrahydrofuran was used as the reaction solvent, the mass ratio of tetrahydrofuran to HMF was 120, the mass ratio of HMF to catalyst was 1.6, the hydrogen pressure was 1.3 MPa, the reaction temperature was 200°C, and the reaction time was 5 h.

0.2 g of the Ni-SCM-14 catalyst from Example 1 above, 0.32 g of HMF, and 38.4 g of tetrahydrofuran were added to an autoclave with stirrer, and 1.3 MPa of hydrogen was charged. The temperature was raised to the preset temperature using a programmed heating mantle, and stirring was performed with a magnetic stirrer. The reaction was carried out at

200°C for 5 h. The reaction liquid was analyzed by gas chromatography/gas chromatography-mass spectrometry to calculate the conversion and selectivities. The HMF conversion was >99%, the selectivity for the product DMF was 88.4%, and the selectivity for the key by-product 5-methylfurfural was 0.2%.

**Example 16**

Tetrahydrofuran was used as the reaction solvent, the mass ratio of tetrahydrofuran to HMF was 120, the mass ratio of HMF to catalyst was 0.9, the hydrogen pressure was 2 MPa, the reaction temperature was 180°C, and the reaction time was 4 h.

0.2 g of the Ni-SCM-14 catalyst from Example 1 above, 0.18 g of HMF, and 21.6 g of tetrahydrofuran were added to an autoclave with stirrer, and 2 MPa of hydrogen was charged. The temperature was raised to the preset temperature using a programmed heating mantle, and stirring was performed with a magnetic stirrer. The reaction was carried out at 180°C for 4 h. The reaction liquid was analyzed by gas chromatography/gas chromatography-mass spectrometry to calculate the conversion and selectivities. The HMF conversion was >99%, the selectivity for the product DMF was 88.2%, and the selectivity for the key by-product 5-methylfurfural was 0.3%.

**Example 17**

Tetrahydrofuran was used as the reaction solvent, the mass ratio of tetrahydrofuran to HMF was 150, the mass ratio of HMF to catalyst was 1.3, the hydrogen pressure was 1.5 MPa, the reaction temperature was 180°C, and the reaction time was 3 h.

0.2 g of the Ni-SCM-14 catalyst from Example 1 above, 0.26 g of HMF, and 39 g of tetrahydrofuran were added to an autoclave with stirrer, and 1.5 MPa of hydrogen was charged. The temperature was raised to the preset temperature using a programmed heating mantle, and stirring was performed with a magnetic stirrer. The reaction was carried out at 180°C for 3 h. The reaction liquid was analyzed by gas chromatography/gas chromatography-mass spectrometry to calculate the conversion and selectivities. The HMF conversion was >99%, the selectivity for the product DMF was 88.1%, and the selectivity for the key by-product 5-methylfurfural was 0.2%.

**Example 18**

Tetrahydrofuran was used as the reaction solvent, the mass ratio of tetrahydrofuran to HMF was 150, the mass ratio of HMF to catalyst was 0.6, the hydrogen pressure was 1 MPa, the reaction temperature was 180°C, and the reaction time was 3 h.

0.2 g of the Ni-SCM-14 catalyst from Example 1 above, 0.12 g of HMF, and 18 g of tetrahydrofuran were added to an autoclave with stirrer, and 1 MPa of hydrogen was charged. The temperature was raised to the preset temperature using a programmed heating mantle, and stirring was performed with a magnetic stirrer. The reaction was carried out at 180°C for 3 h. The reaction liquid was analyzed by gas chromatography/gas chromatography-mass spectrometry to calculate the conversion and selectivities. The HMF conversion was >99%, the selectivity for the product DMF was 89.2%, and the selectivity for the key by-product 5-methylfurfural was 0.3%.

**Example 19**

Tetrahydrofuran was used as the reaction solvent, the mass ratio of tetrahydrofuran to HMF was 100, the mass ratio of HMF to catalyst was 0.8, the hydrogen pressure was 1 MPa, the reaction temperature was 180°C, and the reaction time was 4 h.

0.2 g of the Ni-SCM-14 catalyst from Example 1 above, 0.16 g of HMF, and 16 g of tetrahydrofuran were added to an autoclave with stirrer, and 1 MPa of hydrogen was charged. The temperature was raised to the preset temperature using a programmed heating mantle, and stirring was performed with a magnetic stirrer. The reaction was carried out at 180°C for 4 h. The reaction liquid was analyzed by gas chromatography/gas chromatography-mass spectrometry to calculate the conversion and selectivities. The HMF conversion was >99%, the selectivity for the product DMF was 89.3%, and the selectivity for the key by-product 5-methylfurfural was 0.2%.

**Example 20**

Tetrahydrofuran was used as the reaction solvent, the mass ratio of tetrahydrofuran to HMF was 100, the mass ratio of HMF to catalyst was 1.5, the hydrogen pressure was 2 MPa, the reaction temperature was 180°C, and the reaction time was 6 h.

0.2 g of the Ni-SCM-14 catalyst from Example 1 above, 0.3 g of HMF, and 30 g of tetrahydrofuran were added to

an autoclave with stirrer, and 2 MPa of hydrogen was charged. The temperature was raised to the preset temperature using a programmed heating mantle, and stirring was performed with a magnetic stirrer. The reaction was carried out at 180°C for 6 h. The reaction liquid was analyzed by gas chromatography/gas chromatography-mass spectrometry to calculate the conversion and selectivities. The HMF conversion was >99%, the selectivity for the product DMF was 88.7%, and the selectivity for the key by-product 5-methylfurfural was 0.3%.

**Example 21**

Tetrahydrofuran was used as the reaction solvent, and 5-hydroxymethylfurfural (HMF) and 5-ethoxymethylfurfural (EMF) were used together as reaction substrates. The mass ratio of tetrahydrofuran to the reaction substrates was 100, the mass ratio of the substrates to the catalyst was 1, the hydrogen pressure was 1.5 MPa, the reaction temperature was 190°C, and the reaction time was 3 h.

0.2 g of the catalyst from Example 1 above, 0.09 g of HMF, 0.11 g of EMF, and 20 g of tetrahydrofuran were added to an autoclave with stirrer, and 1.5 MPa of hydrogen was charged. The temperature was raised to the preset temperature using a programmed heating mantle, and stirring was performed with a magnetic stirrer. The reaction was carried out at 190°C for 3 h. The reaction liquid was analyzed by gas chromatography/gas chromatography-mass spectrometry to calculate the conversions and selectivities. The conversions of HMF and EMF were both >99%, the selectivity for the product DMF was 84.3%, and the selectivity for the key by-product 5-methylfurfural was 0.3%.

**Example 22**

0.2 g of the catalyst from Example 1 above, 0.09 g of 5-hydroxymethylfurfural, 0.11 g of 5-butoxymethylfurfural, and 20 g of tetrahydrofuran were added to an autoclave with stirrer, and 1.5 MPa of hydrogen was charged. The temperature was raised to the preset temperature using a programmed heating mantle, and stirring was performed with a magnetic stirrer. The reaction was carried out at 190°C for 3 h. The reaction liquid was analyzed by gas chromatography/gas chromatography-mass spectrometry to calculate the conversions and selectivities. The conversions of HMF and butoxymethylfurfural were both >99%, the selectivity for the product DMF was 83.5%, and the selectivity for the key by-product 5-methylfurfural was 0.2%.

To more intuitively describe the reaction conditions and results of the above Examples 13-22, the various parameters and results are listed in Table 2.

Table 2. Catalytic performance results of Examples 13-22

| Ex. No. | Mass Ratio of Organic Solvent to Substrate | Mass Ratio of Substrate to Catalyst | Hydrogen Pressure (MPa) | Reaction Temperature (°C) | Reaction Time (h) | Conversion (%) | DMF Selectivity (%) | 5-Methylfurfural Selectivity (%) |
|---|---|---|---|---|---|---|---|---|
| 7 | 100 | 1 | 1.5 | 190 | 3 | >99 | 90.1 | 0.2 |
| 13 | 80 | 2 | 2.5 | 170 | 6 | >99 | 87.5 | 0.3 |
| 14 | 80 | 0.8 | 1 | 190 | 2 | >99 | 89.1 | 0.1 |
| 15 | 120 | 1.6 | 1.3 | 200 | 5 | >99 | 88.4 | 0.2 |
| 16 | 120 | 0.9 | 2 | 180 | 4 | >99 | 88.2 | 0.3 |
| 17 | 150 | 1.3 | 1.5 | 180 | 3 | >99 | 88.1 | 0.2 |
| 18 | 150 | 0.6 | 1 | 180 | 3 | >99 | 89.2 | 0.3 |
| 19 | 100 | 0.8 | 1 | 180 | 4 | >99 | 89.3 | 0.2 |
| 20 | 100 | 1.5 | 2 | 180 | 6 | >99 | 88.7 | 0.3 |
| 21 | 100 | 1 | 1.5 | 190 | 3 | >99 | 84.3 | 0.3 |
| 22 | 100 | 1 | 1.5 | 190 | 3 | >99 | 83.5 | 0.2 |

**Example 23**

Tetrahydrofuran was used as the reaction solvent, the mass ratio of tetrahydrofuran to HMF was 100, the mass

ratio of HMF to catalyst was 1, the hydrogen pressure was 1.5 MPa, the reaction temperature was 190°C, and the reaction time was 3 h.

0.2 g of the catalyst from Example 1 above, 0.2 g of HMF, and 20 g of tetrahydrofuran were added to an autoclave with stirrer, and 1.5 MPa of hydrogen was charged. The temperature was raised to the preset temperature using a programmed heating mantle, and stirring was performed with a magnetic stirrer. The reaction was carried out at 190°C for 3 h. The reaction liquid was analyzed by gas chromatography/gas chromatography-mass spectrometry to calculate the HMF conversion and the selectivities for the product DMF and the key by-product 5-methylfurfural. The used catalyst was washed four times with tetrahydrofuran, dried at 100°C, and then used in the next reaction. A total of 4 cycles were performed. The results are shown in Table 3. The results show that after 4 cycles, the HMF conversion remained above 99%, the DMF selectivity remained above 88.7%, and the selectivity for the key by-product 5-methylfurfural was not higher than 0.3%, indicating that the Ni-SCM-14 catalyst has good recycling stability.

Table 3. Catalyst recycling data

| Number of Recycles | HMF Conversion (%) | DMF Selectivity (%) | 5-Methylfurfural Selectivity (%) |
|---|---|---|---|
| 1 | >99 | 90.1 | 0.2 |
| 2 | >99 | 89.5 | 0.2 |
| 3 | >99 | 89.2 | 0.3 |
| 4 | >99 | 88.7 | 0.3 |

**Example 24**

Tetrahydrofuran was used as the reaction solvent, the reaction substrate was hydroxybenzaldehyde, the mass ratio of tetrahydrofuran to hydroxybenzaldehyde was 120, the mass ratio of hydroxybenzaldehyde to catalyst was 1.2, the hydrogen pressure was 1.2 MPa, the reaction temperature was 195°C, and the reaction time was 6 h.

0.1 g of the Ni-SCM-14 catalyst from Example 1 above, 0.12 g of hydroxybenzaldehyde, and 14.4 g of tetrahydrofuran were added to an autoclave with stirrer, and 1.2 MPa of hydrogen was charged. The temperature was raised to the preset temperature using a programmed heating mantle, and stirring was performed with a magnetic stirrer. The reaction was carried out at 195°C for 6 h. The reaction liquid was analyzed by gas chromatography/gas chromatography-mass spectrometry to calculate the conversion and selectivities. The hydroxybenzaldehyde conversion was >99%, the selectivity for the product methylphenol was 95.1%, and the selectivity for the key by-product p-methylcyclohexanol was 0.3%.

**Comparative Example 1**

An SCM-14 molecular sieve was synthesized according to the preparation method described in Example 1 of CN109081360A, wherein the Si/Ge molar ratio was 3.7. The nickel-containing precursor was nickel nitrate. The mass ratio of Ni (the amount of Ni in nickel nitrate) to the above SCM-14 molecular sieve was 1.0:100. The amount of SCM-14 added was 3.0 g. The mixture was thoroughly ground in a mortar to obtain a uniformly dispersed mixture. The above product was dried in an oven at 100°C for 4 h, then calcined at 550°C for 4 h, and reduced under atmospheric hydrogen conditions at a reduction temperature of 400°C for 3 h, to obtain a Ni-SCM-14 molecular sieve catalyst.

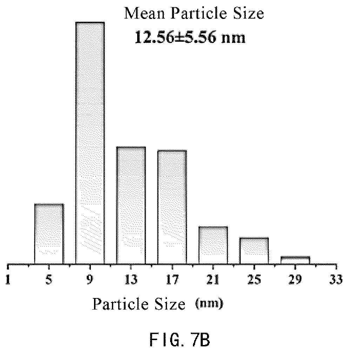

The Si/Ge molar ratio of the sample measured by the inductively coupled plasma atomic emission spectrometer (ICP) was 3.7, and the relative content of Ni was 1.0 wt%. The specific surface area of the sample measured by the BET method was 148 $m^2 \cdot g^{-1}$. The total acid amount calculated from the $NH_3$-TPD pattern of the sample was 173 $\mu mol \cdot g^{-1}$. The Brønsted acid/Lewis acid ratio calculated from the pyridine infrared spectrum of the sample was 0.74. The $Ni/Ni^{2+}$ ratio calculated from the XPS spectrum of the sample was 0.41. The TEM image of the sample is shown in FIG. 7A, showing that the distribution of nickel was uneven, and the mean particle size of nickel particles was 12.6 nm.

Tetrahydrofuran was used as the reaction solvent, the mass ratio of tetrahydrofuran to HMF was 100, the mass ratio of HMF to catalyst was 1, the hydrogen pressure was 1.5 MPa, the reaction temperature was 190°C, and the reaction time was 3 h. 0.2 g of the catalyst from Comparative Example 1 above, 0.2 g of HMF, and 20 g of tetrahydrofuran were added to an autoclave with stirrer, and 1.5 MPa of hydrogen was charged. The temperature was raised to the preset temperature using a programmed heating mantle, and stirring was performed with a magnetic stirrer. The reaction was carried out at 190°C for 3 h. The reaction liquid was analyzed by gas chromatography/gas chromatography-mass spectrometry to calculate the conversion and selectivities. The HMF conversion was >99%, the selectivity for the product DMF was 25.9%, and the selectivity for the key by-product 5-methylfurfural was 2.7%.

**Comparative Example 2**

An SCM-14 molecular sieve was synthesized according to the preparation method described in Example 1 of CN109081360A, wherein the Si/Ge molar ratio was 3.7.

Tetrahydrofuran was used as the reaction solvent, the mass ratio of tetrahydrofuran to HMF was 100, the mass ratio of HMF to catalyst was 1, the hydrogen pressure was 1.5 MPa, the reaction temperature was 190°C, and the reaction time was 3 h. 0.2 g of the above SCM-14 molecular sieve (used as a catalyst), 0.2 g of HMF, and 20 g of tetrahydrofuran were added to an autoclave with stirrer, and 1.5 MPa of hydrogen was charged. The temperature was raised to the preset temperature using a programmed heating mantle, and stirring was performed with a magnetic stirrer. The reaction was carried out at 190°C for 3 h. The reaction liquid was analyzed by gas chromatography/gas chromatography-mass spectrometry to calculate the HMF conversion, which was 7%. The product DMF and the key by-product 5-methylfurfural were not detected.

**Comparative Example 3**

An SCM-14 molecular sieve was synthesized according to the preparation method described in Example 1 of CN109081360A, wherein the Si/Ge molar ratio was 3.7. The nickel-containing precursor was nickel acetylacetonate. The mass ratio of Ni (the amount of Ni in nickel acetylacetonate) to the above SCM-14 molecular sieve was 1.0:100. The amount of SCM-14 added was 3.0 g. The mixture was thoroughly ground in a mortar to obtain a uniformly dispersed mixture. The above product was dried in an oven at 100°C for 4 h, then calcined at 550°C for 4 h, and reduced under atmospheric hydrogen conditions at a reduction temperature of 400°C for 3 h, to obtain a Ni-SCM-14 molecular sieve.

The Si/Ge molar ratio of the sample measured by the inductively coupled plasma atomic emission spectrometer (ICP) was 3.7, and the relative content of Ni was 1.0 wt%. The specific surface area of the sample measured by the BET method was 137 $m^2 \cdot g^{-1}$. The total acid amount calculated from the $NH_3$-TPD pattern of the sample was 161 $\mu mol \cdot g^{-1}$. The Brønsted acid/Lewis acid ratio calculated from the pyridine infrared spectrum of the sample was 0.82. The $Ni/Ni^{2+}$ ratio calculated from the XPS spectrum of the sample was 0.35. According to the TEM image of the sample, the mean particle size of nickel particles was calculated to be 10.3 nm.

Tetrahydrofuran was used as the reaction solvent, the mass ratio of tetrahydrofuran to HMF was 100, the mass ratio of HMF to catalyst was 1, the hydrogen pressure was 1.5 MPa, the reaction temperature was 190°C, and the reaction time was 3 h. 0.2 g of the catalyst from Comparative Example 3 above, 0.2 g of HMF, and 20 g of tetrahydrofuran were added to an autoclave with stirrer, and 1.5 MPa of hydrogen was charged. The temperature was raised to the preset temperature using a programmed heating mantle, and stirring was performed with a magnetic stirrer. The reaction was carried out at 190°C for 3 h. The reaction liquid was analyzed by gas chromatography/gas chromatography-mass spectrometry to calculate the conversion and selectivities. The HMF conversion was >99%, the selectivity for the product DMF was 37.2%, and the selectivity for the key by-product 5-methylfurfural was 3.3%.

**Comparative Example 4**

The method described in Example 1 was repeated, wherein SCM-14 was replaced with ZSM-5 to obtain Ni-ZSM-5, wherein ZSM-5 was a commercially available molecular sieve (Tianjin Nanhua Catalyst Co., Ltd., NKF-5D-38H).

Tetrahydrofuran was used as the reaction solvent, the mass ratio of tetrahydrofuran to HMF was 100, the mass ratio of HMF to catalyst was 1, the hydrogen pressure was 1.5 MPa, the reaction temperature was 190°C, and the reaction time was 3 h. 0.2 g of the above Ni-ZSM-5, 0.2 g of HMF, and 20 g of tetrahydrofuran were added to an autoclave with stirrer, and 1.5 MPa of hydrogen was charged. The temperature was raised to the preset temperature using a programmed heating mantle, and stirring was performed with a magnetic stirrer. The reaction was carried out at 190°C for 3 h. The reaction liquid was analyzed by gas chromatography/gas chromatography-mass spectrometry to calculate the conversion and selectivities. The HMF conversion was 98.5%, the selectivity for the product DMF was 21.6%, and the selectivity for the key by-product 5-methylfurfural was 5.7%.

**Comparative Example 5**

The method described in Example 1 was repeated, wherein SCM-14 was replaced with USY-6 to obtain Ni-USY-6, wherein USY-6 was a commercially available molecular sieve (Sinopec Catalyst Co., Ltd.).

Tetrahydrofuran was used as the reaction solvent, the mass ratio of tetrahydrofuran to HMF was 100, the mass ratio of HMF to catalyst was 1, the hydrogen pressure was 1.5 MPa, the reaction temperature was 190°C, and the reaction time was 3 h. 0.2 g of the above Ni-USY-6, 0.2 g of HMF, and 20 g of tetrahydrofuran were added to an autoclave with stirrer, and 1.5 MPa of hydrogen was charged. The temperature was raised to the preset temperature using a programmed heating mantle, and stirring was performed with a magnetic stirrer. The reaction was carried out at 190°C for 3 h. The

reaction liquid was analyzed by gas chromatography/gas chromatography-mass spectrometry to calculate the conversion and selectivities. The HMF conversion was 70.1%, the selectivity for the product DMF was 11.2%, and the selectivity for the key by-product 5-methylfurfural was 8.5%.

Table 4A. Catalytic evaluation results of the catalysts in Comparative Examples 1-5

| CE. No. | Catalyst | Ni Precursor | HMF Conversion (%) | DMF Selectivity (%) | 5-Methylfurfural Selectivity (%) |
|---|---|---|---|---|---|
| 1 | Ni-SCM-14 | Nickel nitrate | >99 | 25.9 | 2.7 |
| 2 | SCM-14 | - | 7 | 0 | 0 |
| 3 | Ni-SCM-14 | Nickel acetylacetonate | >99 | 37.2 | 3.3 |
| 4 | Ni-ZSM-5 | Nickelocene | 98.5 | 21.6 | 5.7 |
| 5 | Ni-USY-6 | Nickelocene | 70.1 | 11.2 | 8.5 |

**Comparative Examples 6-9**

The following hydrogenation catalysts were used:

Pd/C (Acros Organics, 195020100, 5% Pd);
Ru/C (Alfa Aesar, 044338, 5% Ru);
Ni-Co/$SiO_2$ and Ni-Co/$Al_2O_3$ (synthesized according to P. Yang et al., J. Energy. Chem. 25 (2016) 1015-1020, Ni content: 2 wt%, Co content: 20 wt%).

Tetrahydrofuran was used as the reaction solvent, the mass ratio of tetrahydrofuran to HMF was 100, the mass ratio of HMF to catalyst was 1, the hydrogen pressure was 1.5 MPa, the reaction temperature was 190°C, and the reaction time was 3 h.

0.2 g of each of the above hydrogenation catalysts, 0.2 g of HMF, and 20 g of tetrahydrofuran were added to an autoclave with stirrer, and 1.5 MPa of hydrogen was charged. The temperature was raised to the preset temperature using a programmed heating mantle, and stirring was performed with a magnetic stirrer. The reaction was carried out at 190°C for 3 h. The reaction liquid was analyzed by gas chromatography/gas chromatography-mass spectrometry to calculate the HMF conversion and the selectivities for the product DMF and the key by-product 5-methylfurfural, which are shown in Table 4B.

Table 4B. Catalytic evaluation results of the catalysts in Comparative Examples 6-9

| CE. No. | Catalyst | HMF Conversion (%) | DMF Selectivity (%) | 5-Methylfurfural Selectivity (%) |
|---|---|---|---|---|
| 6 | Pd/C | >99 | 80.2 | 5.3 |
| 7 | Ru/C | >99 | 63.5 | 16.2 |
| 8 | Ni-Co/$SiO_2$ | >99 | 81.3 | 3.5 |
| 9 | Ni-Co/$Al_2O_3$ | >99 | 65.4 | 7.7 |

**Comparative Example 10**

An SCM-14 molecular sieve was synthesized according to the preparation method described in Example 1 of CN109081360A, wherein the Si/Ge molar ratio was 3.7. The nickel-containing precursor was nickelocene. The mass ratio of Ni (the amount of Ni in the organometallic precursor) to the above SCM-14 molecular sieve was 0.3:100. The amount of SCM-14 added was 3.0 g. The mixture was thoroughly ground in a mortar to obtain a uniformly dispersed mixture. The above product was dried in an oven at 100°C for 4 h, then calcined at 550°C for 4 h, and reduced under atmospheric hydrogen conditions at a reduction temperature of 400°C for 3 h, to obtain a Ni-SCM-14 molecular sieve catalyst.

The Si/Ge molar ratio of the sample measured by the inductively coupled plasma atomic emission spectrometer (ICP) was 3.7, and the relative content of Ni was 0.30 wt%. The specific surface area of the sample measured by the BET method was 143 $m^2 \cdot g^{-1}$. The total acid amount calculated from the $NH_3$-TPD pattern of the sample was 130 $\mu mol \cdot g^{-1}$. The Brønsted acid/Lewis acid ratio calculated from the pyridine infrared spectrum of the sample was 0.43. According to the TEM image of the sample, the mean particle size of nickel particles was calculated to be 6.3 nm.

The XPS spectrum of the sample showed that the molar ratio of metallic Ni to $Ni^{2+}$ (calculated from the ratio of the two peak areas) ($Ni/Ni^{2+}$ ratio) was 0.01.

Tetrahydrofuran was used as the reaction solvent, the mass ratio of tetrahydrofuran to HMF was 100, the mass ratio of HMF to catalyst was 1, the hydrogen pressure was 1.5 MPa, the reaction temperature was 190°C, and the reaction time was 3 h. 0.2 g of the catalyst from Comparative Example 10 above, 0.2 g of HMF, and 20 g of tetrahydrofuran were added to an autoclave with stirrer, and 1.5 MPa of hydrogen was charged. The temperature was raised to the preset temperature using a programmed heating mantle, and stirring was performed with a magnetic stirrer. The reaction was carried out at 190°C for 3 h. The reaction liquid was analyzed by gas chromatography/gas chromatography-mass spectrometry to calculate the conversion and selectivities. The HMF conversion was >99%, the selectivity for the product DMF was 30.3%, and the selectivity for the key by-product 5-methylfurfural was 3.9%.

**Comparative Example 11**

An SCM-14 molecular sieve was synthesized according to the preparation method described in Example 1 of CN109081360A, wherein the Si/Ge molar ratio was 3.7. The nickel-containing precursor was nickelocene. The mass ratio of Ni (the amount of Ni in the organometallic precursor) to the above SCM-14 molecular sieve was 9:100. The amount of SCM-14 added was 3.0 g. The mixture was thoroughly ground in a mortar to obtain a uniformly dispersed mixture. The above product was dried in an oven at 100°C for 4 h, then calcined at 550°C for 4 h, and reduced under atmospheric hydrogen conditions at a reduction temperature of 400°C for 3 h, to obtain a Ni-SCM-14 molecular sieve.

The Si/Ge molar ratio of the sample measured by the inductively coupled plasma atomic emission spectrometer (ICP) was 3.7, and the relative content of Ni was 8.3 wt%. The specific surface area of the sample measured by the BET method was 132 $m^2 \cdot g^{-1}$. The total acid amount calculated from the $NH_3$-TPD pattern of the sample was 205 $\mu mol \cdot g^{-1}$. The Brønsted acid/Lewis acid ratio calculated from the pyridine infrared spectrum of the sample was 0.69. According to the TEM image of the sample, the mean particle size of nickel particles was calculated to be 4.3 nm.

The XPS spectrum of the sample showed that the molar ratio of metallic Ni to $Ni^{2+}$ (calculated from the ratio of the two peak areas) ($Ni/Ni^{2+}$ ratio) was 0.19.

Tetrahydrofuran was used as the reaction solvent, the mass ratio of tetrahydrofuran to HMF was 100, the mass ratio of HMF to catalyst was 1, the hydrogen pressure was 1.5 MPa, the reaction temperature was 190°C, and the reaction time was 3 h. 0.2 g of the catalyst from Comparative Example 11 above, 0.2 g of HMF, and 20 g of tetrahydrofuran were added to an autoclave with stirrer, and 1.5 MPa of hydrogen was charged. The temperature was raised to the preset temperature using a programmed heating mantle, and stirring was performed with a magnetic stirrer. The reaction was carried out at 190°C for 3 h. The reaction liquid was analyzed by gas chromatography/gas chromatography-mass spectrometry to calculate the conversion and selectivities. The HMF conversion was >99%, the selectivity for the product DMF was 41.6%, and the selectivity for the key by-product 5-methylfurfural was 8.1%.

The specific embodiments of the present disclosure have been described in detail above, however, the present disclosure is not limited thereto. Within the technical concept of the present disclosure, various simple modifications can be made to the technical solution of the present disclosure, including combining various technical features in any other suitable manner. These simple modifications and combinations should also be regarded as the content disclosed in the present disclosure and fall within the protection scope of the present disclosure.

**Claims**

1. A Ni-SCM-X molecular sieve catalyst, wherein X is 14 or 15; wherein Ni comprises metallic Ni and $Ni^{2+}$, wherein the molar ratio of $Ni/Ni^{2+}$ is in a range of 0.02-0.15, preferably 0.05-0.12.

2. The catalyst according to claim 1, **characterized in that** the catalyst comprises nickel particles, wherein the mean particle size of the nickel particles is in a range of 2.0-8.0 nm, preferably 3.0-7.0 nm, more preferably 3.0-6.0 nm.

3. The catalyst according to any one of claims 1-2, **characterized in that**, in the catalyst, based on the mass of the SCM-X molecular sieve, the content of Ni, calculated as elemental Ni, is in a range of 0.2 wt%-5.0 wt%, preferably 0.5 wt%-3.0 wt%.

4. The catalyst according to any one of claims 1-3, **characterized in that**, in the Ni-SCM-X molecular sieve catalyst, the Si/Ge molar ratio of the SCM-X molecular sieve is in a range of 1.5-8, preferably 2-7.

5. The catalyst according to any one of claims 1-4, **characterized in that** the total specific surface area of the Ni-SCM-X molecular sieve catalyst is in a range of 90-370 $m^2 \cdot g^{-1}$, preferably 100-350 $m^2 \cdot g^{-1}$; and/or

the total acid amount of the Ni-SCM-X molecular sieve catalyst is in a range of 130-220 $\mu mol \cdot g^{-1}$, preferably 150-200 $\mu mol \cdot g^{-1}$; and/or
the Brønsted acid/Lewis acid ratio of the Ni-SCM-X molecular sieve catalyst is in a range of 0.3-0.8, preferably 0.4-0.6; and/or,
the Ni-SCM-X molecular sieve catalyst is used in a reaction for preparing 2,5-dimethylfuran from 5-hydroxymethylfurfural and/or 5-(C1-C20 alkoxymethyl)furfural, preferably the 5-(C1-C20 alkoxymethyl)furfural is 5-(C1-C8 alkoxymethyl)furfural, more preferably 5-(C1-C5 alkoxymethyl)furfural.

**6.** A method for preparing the Ni-SCM-X molecular sieve catalyst according to any one of claims 1-5, comprising:

mixing an SCM-X molecular sieve with a nickel-containing precursor to obtain a mixture, and
calcining and reducing the mixture to obtain the Ni-SCM-X molecular sieve catalyst.

**7.** The method according to claim 6, **characterized in that** the nickel-containing precursor is selected from nickel precursors containing an organic ligand, preferably the organic ligand is selected from organic ligands containing a five-membered carbocyclic ring, a six-membered carbocyclic ring, or an eight-membered carbocyclic ring, more preferably the five-membered carbocyclic ring is a cyclopentadiene ring, the six-membered carbocyclic ring is a benzene ring, and the eight-membered carbocyclic ring is a cyclooctadiene ring; preferably the nickel precursor containing an organic ligand is selected from at least one of nickelocene, bis-(1,5-cyclooctadiene) nickel, bis(tetramethylcyclopentadienyl) nickel, 1,2-bis(diphenylphosphino)ethane nickel chloride and bis(triphenylphosphine) nickel chloride, more preferably nickelocene.

**8.** The method according to claim 6 or 7, **characterized in that** the mass ratio of Ni in the nickel-containing precursor to the SCM-X molecular sieve is in a range of 0.2-5.0:100, preferably 0.5-3.0:100.

**9.** The method according to any one of claims 6-8, **characterized in that** the mixing is physical mixing; preferably the mixing is grinding or ball milling; and/or
the method further comprises drying the mixture before the calcination and reduction; preferably the conditions for drying include drying at 50-130°C for 2-8 h.

**10.** The method according to any one of claims 6-9, **characterized in that** the conditions for calcination include: calcining at 300-650°C for 1-12 h, and the calcination atmosphere is an oxygen-containing gas, preferably the calcination atmosphere is oxygen, air, or a mixed gas of oxygen and air; and/or,
the reduction is carried out under a hydrogen atmosphere, the reduction temperature is in a range of 300-500°C, preferably 350-450°C; and the reduction time is in a range of 1-8 h, preferably 2-6 h.

**11.** A method for preparing 2,5-dimethylfuran, comprising: in the presence of the catalyst according to any one of claims 1-5, subjecting 5-hydroxymethylfurfural and/or 5-(C1-C20 alkoxymethyl)furfural to hydrogenolysis in an organic solvent to prepare 2,5-dimethylfuran, preferably the 5-(C1-C20 alkoxymethyl)furfural is 5-(C1-C8 alkoxymethyl) furfural, more preferably 5-(C1-C5 alkoxymethyl)furfural.

**12.** The method according to claim 11, **characterized in that** the organic solvent comprises at least one of C1-C5 alcohols, cyclic ether compounds, γ-valerolactone, methyl isobutyl ketone, and acetone, preferably the C1-C5 alcohol is selected from methanol, ethanol, n-propanol, n-butanol, isobutanol, isopropanol, isoamyl alcohol, ethylene glycol, propylene glycol and glycerol, and the cyclic ether compound is selected from pyran, 2-methyltetrahydrofuran, 2,5-dimethyltetrahydrofuran, tetrahydrofuran and 1,4-dioxane; preferably the organic solvent comprises at least one of n-butanol, acetone, 1,4-dioxane, γ-valerolactone, methyl isobutyl ketone, and tetrahydrofuran, more preferably the organic solvent comprises tetrahydrofuran.

**13.** The method according to claim 11 or 12, **characterized in that** the hydrogenolysis is carried out under the following conditions: a reaction temperature in a range of 130-220°C, preferably 150-200°C; a reaction time in a range of 1-12 h, preferably 2-10 h; and a hydrogen pressure in a range of 0.5-4 MPa, preferably 1-3 MPa; and/or,

the mass ratio of 5-hydroxymethylfurfural and/or 5-(C1-C20 alkoxymethyl)furfural to the catalyst is in a range of 0.2-5:1, preferably 0.5-4:1; and/or,
the mass ratio of the organic solvent to 5-hydroxymethylfurfural and/or 5-(C1-C20 alkoxymethyl)furfural is in a range of 40-400:1, preferably 50-300:1.

**14.** Use of the catalyst according to any one of claims 1-5 or the catalyst prepared by the preparation method according to any one of claims 6-10 as a catalyst in a reaction for preparing 2,5-dimethylfuran from 5-hydroxymethylfurfural and/or 5-(C1-C20 alkoxymethyl)furfural, preferably the 5-(C1-C20 alkoxymethyl)furfural is 5-(C1-C8 alkoxymethyl)furfural, more preferably 5-(C1-C5 alkoxymethyl)furfural.

Ni-SCM-14
Intensity (a.u.)
10
20
30
40
50
60
70
2θ(°)

FIG. 1

Ni-SCM-14
Intensity (a.u.)
100
200
300
400
500
Bed Temperature (°C)

FIG. 2

Ni-SCM-14
Intensity (a.u.)
1560
1540
1520
1500
1480
1460
1440
1420
Wavenumber (cm-1)

FIG. 3

Ni 2p3/2
Ni2+ 93.0%
855.6
Ni0 7.0%
852.0
Intensity (a.u.)
868
866
864
862
860
858
856
854
852
850
Binding Energy (eV)

FIG. 4

50 nm

FIG. 5A

Mean Particle Size
3.48±0.55 nm
2
2.5
3
3.5
4
4.5
5
5.5
Particle Size (nm)

FIG. 5B

Ni-SCM-15
Intensity (a.u.)
0
10
20
30
40
50
2θ(°)

FIG. 6

100 nm

FIG. 7A

Mean Particle Size
12.56±5.56 nm
1
5
9
13
17
21
25
29
33
Particle Size (nm)

FIG. 7B

**INTERNATIONAL SEARCH REPORT**

International application No.
**PCT/CN2024/122744**

**A. CLASSIFICATION OF SUBJECT MATTER**

B01J29/ 00(2006.01)i; B01J37/ 04(2006.01)i; C07D307/36 (2006.01)i

According to International Patent Classification (IPC) or to both national classification and IPC

**B. FIELDS SEARCHED**

Minimum documentation searched (classification system followed by classification symbols)

IPC: B01J29/ -、B01J37/ -、C07D307/-

Documentation searched other than minimum documentation to the extent that such documents are included in the fields searched

Electronic data base consulted during the international search (name of data base and, where practicable, search terms used)

CNABS, CNTXT, CNKI, ENTXT, VEN, Web of Science: 镍, SCM-14, SCM-15, 硅锗, 2, 5-二甲基呋喃, Ni, nickel, germanosilicate, 2, 5-dimethylfuran, DMF

**C. DOCUMENTS CONSIDERED TO BE RELEVANT**

| Category* | Citation of document, with indication, where appropriate, of the relevant passages | Relevant to claim No. |
|---|---|---|
| A | CN 116003202 A (CHINA PETROLEUM & CHEMICAL CORP. et al.) 25 April 2023 (2023-04-25) claims 1-10 | 1-14 |
| A | CN 106279075 A (CHINA UNIVERSITY OF PETROLEUM, BEIJING) 04 January 2017 (2017-01-04) entire document | 1-14 |
| A | CN 109081359 A (CHINA PETROLEUM & CHEMICAL CORP. et al.) 25 December 2018 (2018-12-25) entire document | 1-14 |
| A | CN 111875566 A (HUNAN NORMAL UNIVERSITY) 03 November 2020 (2020-11-03) entire document | 1-14 |
| A | US 2016339414 A1 (COUNCIL OF SCIENTIFIC AND INDUSTRIAL RESEARCH) 24 November 2016 (2016-11-24) entire document | 1-14 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 December 2024** | **09 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/122744**

| Patent document cited in search report | Publication date (day/month/year) | Patent family member(s) | Publication date (day/month/year) |
|---|---|---|---|
| CN 116003202 A | 25 April 2023 | None | |
| CN 106279075 A | 04 January 2017 | None | |
| CN 109081359 A | 25 December 2018 | SG 11201912140 XA | 30 January 2020 |
| | | DK 3640207 T3 | 11 April 2022 |
| | | EP 3640207 A1 | 22 April 2020 |
| | | EP 3640207 A4 | 03 March 2021 |
| | | EP 3640207 B1 | 09 February 2022 |
| | | ES 2910829 T3 | 13 May 2022 |
| | | KR 20200018453 A | 19 February 2020 |
| | | KR 102356362 B1 | 27 January 2022 |
| | | PT 3640207 T | 14 April 2022 |
| | | BR 112019026405 A2 | 21 July 2020 |
| | | WO 2018227850 A1 | 20 December 2018 |
| | | US 2020188891 A1 | 18 June 2020 |
| | | US 10974967 B2 | 13 April 2021 |
| | | JP 2020523271 A | 06 August 2020 |
| | | JP 7094992 B2 | 04 July 2022 |
| CN 111875566 A | 03 November 2020 | None | |
| US 2016339414 A1 | 24 November 2016 | EP 3097087 A1 | 30 November 2016 |
| | | EP 3097087 B1 | 27 December 2017 |
| | | WO 2015111078 A1 | 30 July 2015 |
| | | US 9757713 B2 | 12 September 2017 |
| | | CA 2937511 A1 | 30 July 2015 |
| | | CA 2937511 C | 05 July 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 114832833 A
- CN 108654693 B
- CN 106279075 B
- CN 109081360 A
- WO 2018227849 A1
- CN 109081359 A
- WO 2018227850 A1


### Non-patent literature cited in the description


- **P. YANG et al.** *J. Energy. Chem.*, 2016, vol. 25, 1015-1020